# EUROPEAN PATENT APPLICATION

(11) **EP 1 598 607 A2**
(43) Date of publication of application: **23.11.2005**
(21) Application number: 05101746.5
(22) Date of filing: 07.03.2005
(51) Int. Cl.: F24F 12/00, F28D 9/00

(54) **Ventilator**

(30) Priority: 14.05.2004 KR 2004034154
(71) Applicant: LG ELECTRONICS INC., Seoul 150-875 (KR)
(72) Inventor: LEE, Jung Woo, SEOUL (KR); CHIN, Sim Won, Gyeonggi-do (KR); LEE, Sang Yeul, 327-23 Gasan-dong, Geumchun-gu, SEOUL (KR)
(74) Representative: Ekström, Nils

(57) **Abstract**

The present invention relates to a ventilator. Channels are horizontally extended in the interior of a total heat exchanger. The channels are vertically stacked for thereby enhancing the efficiency of heat exchange.

## Description

### BACKGROUND OF THE INVENTION

### 1. Field of the Invention

The present invention relates to a ventilator, and in particular to an improved ventilator that allows to form a smaller heat exchanger by enhancing a heat exchange efficiency of supplied air and exhausted air in a heat exchanger in which the heat exchange is performed by making the supplied air and exhausted air to crossed each other.

### 2. Description of the Background Art

Generally, in a certain limited space in which air is not well circulated, and various kinds of lives breath together, the amount of oxygen is gradually decreased, and the amount of carbon dioxide is gradually increased. When a person stays in a limited narrow space or a space with many persons therein, it is needed to regularly change the indoor air with external fresh air. In this case, a ventilator is generally used.

The conventional ventilator adapts a method of forcibly discharging indoor air to the outside using one blower. However, in the case that the indoor air is forcibly discharged using one blower, since the indoor warm air or hot air are directly discharged to the outside without passing through a certain filter, the cost is additionally needed for warming or hearing the indoor air to a desired temperature.

In order to overcome the above problems, a ventilator adapting a total heat exchange method has been developed. In the above ventilator, the supplied outdoor air is heat-exchanged with the discharged indoor air.

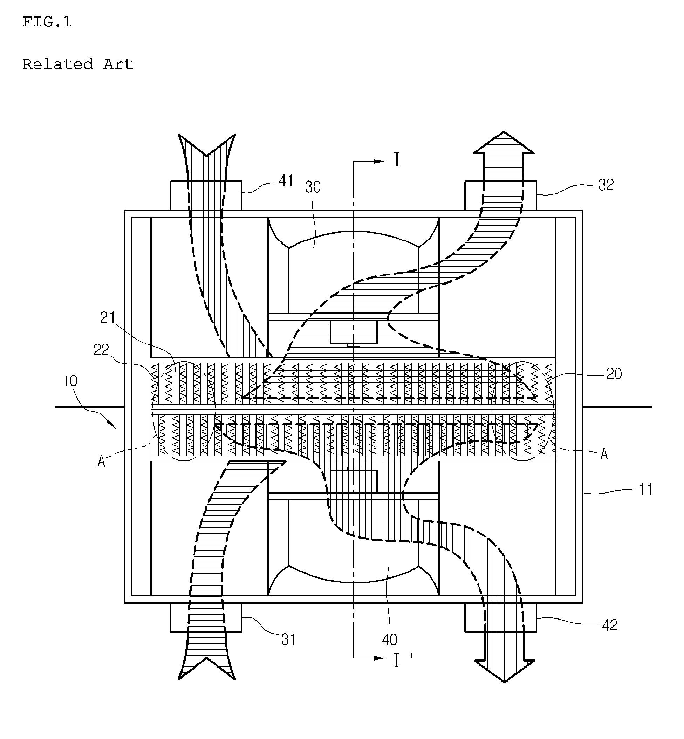

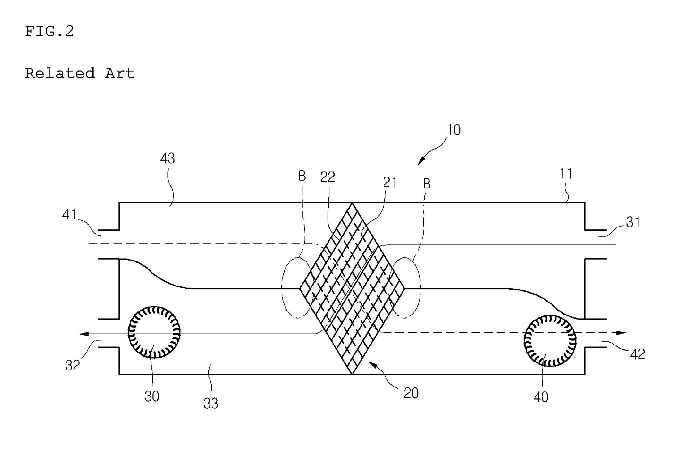

Figure 1 is a plane view of a ventilator for describing the operation of a conventional ventilator, and Figure 2 is a cross sectional view taken along line I-I' of Figure 2.

As shown in Figures 1 and 2, a conventional ventilator 10 includes a casing 11 forming an outer construction, an air supply suction part 31 formed in one surface of the casing 11 for sucking outdoor air, a suction duct 33 for sucking outdoor air sucked through the air supply suction part 31, and an air supply discharge part 32 for discharging outdoor air into an indoor. In addition, an exhaust suction part 41 is formed in one surface of the casing 11 for sucking indoor air. The indoor air sucked through the exhaust suction part 41 is flown by an exhaust duct 43. An exhaust discharge part 42 discharges the indoor air to the outdoor. In addition, there are further provided a suction fan 30 installed in the air supply suction part 31 and the air supply discharge part 32 for sucking indoor air, and an exhaust fan 40 installed in a flow path connecting the exhaust part 41 and the exhaust discharge part 42 for sucking indoor air.

In addition, there is further provided a total heat exchanger capable of achieving heat exchange in such a manner that outdoor air and indoor are crossed with each other. In detail, the total heat exchanger 20 includes a suction air flow path 21 for flowing outdoor air, and an exhaust flow path 22 for flowing indoor air. Here, since the suction air flow path 21 and the exhaust flow path 22 are alternately formed in a layer structure with a partition wall therebetween, that outdoor air and indoor air are not mixed during heat exchange.

Namely, in the conventional ventilator, the heat exchange is achieved in such a manner that the outdoor and indoor air inputted through the outdoor and indoor sides are not mixed with each other when they pass through the suction air flow path 21 and the exhaust flow path 22 of the total heat exchanger 20. In detail, the total heat exchanger 20 has a lateral cross section of a diamond shape and is longitudinally extended in a horizontal direction. In addition, the sucked outdoor and indoor air are inputted along a lower slanted surface of the total heat exchanger 20 and are discharged along the upper side slanted surface. The sucked outdoor and indoor are inputted along the upper side slanted surface and are discharged along the lower side slanted surface.

However, since the total heat exchanger 20 is arranged in a diamond shape, the conventional ventilator has the following problems.

Since there is a limit in the vertical height of the ventilator, the cross section dimension of the total heat exchanger has a certain limit. Therefore, it is impossible to make the cross section of the total heat exchange larger. In addition, in the case that the length of the total heat exchanger is extended in order to compensate the limit of the cross section of the total heat exchanger, the size of the total heat exchanger is increased. In addition, as the length of the total heat exchanger is extended, the airflow path passing through the total heat exchanger is bent at many points, so that the resistance of the flow path is increased.

Since air is inputted through the lower side and is discharged through the upper side, in other words, since the flow path is bent in the upper and lower directions, a multiple layer flow section B is formed in the flow path at both sides of the inlet and outlet of the total heat exchanger. In this case, the resistance of the flow path is also increased.

The air inputted eccentrically inputted in the left or right direction in the total heat exchanger does not reach at the opposite suction air flow path 21 or the exhaust flow path 22, so that a dead zone is formed wherein the efficiency of heat exchange is sharply decreased therein. Since the heat exchange is not actually performed in the dead zone, the efficiency of the heat exchange of the total heat exchanger 20 is decreased.

### SUMMARY OF THE INVENTION

Accordingly, it is an object of the present invention to provide a ventilator structure capable of overcoming the problems encountered in the conventional art.

It is another object of the present invention to provide a ventilator structure capable of minimizing the dimension of a ventilator by improving a structure of a total heat exchanger that performs heat exchanger by outdoor air and indoor air.

It is further another object of the present invention to provide a ventilator capable of decreasing a resistance in a flow path in such a manner that an airflow path is simplified, and a multiple layer flow section in a flow path is removed. In addition, a dead zone is not formed in the interior of a total heat exchanger in such a manner that air is uniformly flown in the entire flow paths in a horizontal direction of a total heat exchanger.

It is still further another object of the present invention to provide a ventilator capable of significantly enhancing a heat exchange efficiency of a total heat exchanger and capable of enhancing an efficiency in use of a space in such a manner that a ventilator is conveniently installed in a narrow installation space.

It is still further another object of the present invention to provide a ventilator in which a multiple layer flow section does not occur in a flow path, and a dead zone is not formed in a flow path, for thereby increasing a heat exchange efficiency of a total heat exchanger.

To achieve the above objects, there is provided a ventilator, comprising a casing; a flow path that is formed in the interior of the casing wherein indoor air and/or outdoor air are sucked and discharged through the flow path; and a total heat exchanger that is connected with the flow path wherein the indoor air and/or outdoor flow through a plurality of channels horizontally extended, and the upper and lower sides of the total heat exchanger are blocked.

### BRIEF DESCRIPTION OF THE DRAWINGS

The present invention will become better understood with reference to the accompanying drawings which are given only by way of illustration and thus are not limitative of the present invention, wherein;
Figure 1 is a plane view of a ventilator for describing the operation of a conventional ventilator;
Figure 2 is a cross sectional view taken along line I-I' of Figure 2;
Figure 3 is a schematic perspective view of a ventilator according to a first embodiment of the present invention;
Figure 4 is a perspective view illustrating a total heat exchanger according to the present invention;
Figure 5 is an enlarged view of the portion C of Figure 4;
Figure 6 is a plane view illustrating the operation of the ventilator according to the present invention;
Figure 7 is a plane view of a ventilator according to a second embodiment of the present invention;
Figure 8 is a plane view of a ventilator according to a third embodiment of the present invention; and
Figure 9 is a plane view illustrating a ventilator according to a fourth embodiment of the present invention.

### DETAILED DESCRIPTION OF THE PREFERRED EMBODIMENTS

The preferred embodiments of the present invention will be described with reference to the accompanying drawings.

### [First embodiment]

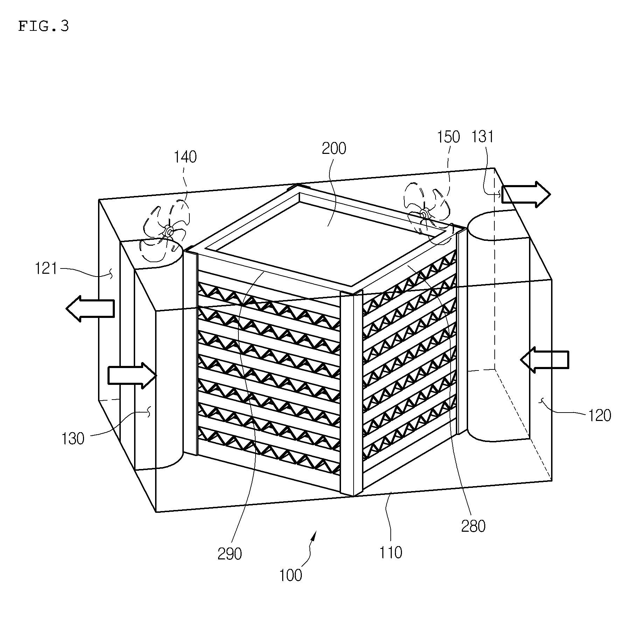

Figure 3 is a schematic perspective view of a ventilator according to a first embodiment of the present invention.

As shown in Figure 3, the ventilator 100 according to the present invention includes a casing 110 that forms an outer construction of the ventilator and protects inner elements, the suction air suction part 120 that is provided in one surface of the casing 110 and sucks outdoor air, and an air suction discharge part 121 that is provided in the other surface of the casing 110 so that air sucked through the suction air suction part 120 is discharged to the indoor. In addition, there are further provided an exhaust suction part 130 that is provided in one surface of the casing 110 and sucks indoor air, and an exhaust discharge part 131 that is provided in the other surface of the casing 110 so that the indoor air sucked through the exhaust suction part 130 is discharged to the outdoor. In addition, a total heat exchanger 200 is provided in a center portion of the ventilator 100 wherein the air inputted into the interior of the ventilator 100 through the suction air suction part 120 and the exhaust suction part 130 is crossed and heat-exchanged. A suction fan 140 and an exhaust fan 150 are installed in the interior of the ventilator 100, so that the outdoor air and indoor air forcibly flow thereby.

A plurality of stacked channels are formed in the total heat exchanger 200 in the upper and lower directions. The proceeding directions of the flow path in which the channels are formed are crossed with each other in the vertical direction. Therefore, the flow path for the air sucked and the flow path for the air exhausted are not mixed. In addition, since the channels are stacked in the total heat exchanger 200 in the upper and lower directions, it is easy to expand the area of heat exchange. The air flowing in the flow path does not turn in the upper and lower directions, but turn only in the horizontal direction, so that a flow path resistance by the flow of air is very small. Therefore, the resistance in airflow is very small, and the efficiency of the heat exchange is enhanced.

The operation of the ventilator according to the present invention will be described.

When power is supplied to the ventilator 100, the suction fan 140 for sucking outdoor air is operated, and the exhaust fan 150 for sucking indoor air is operated. In addition, the outdoor air and indoor air are inputted into the ventilator 100 through the suction air suction part 120 based on the operation of the suction fan 140. The indoor air is inputted into the interior of the ventilator 100 through the exhaust suction part 130 based on the operation of the exhaust fan 150. In addition, the indoor air and outdoor air inputted into the interior of the ventilator 100 perform a heat exchange operation, while passing through the total heat exchanger 200. At this time, since the flow path of the indoor air is filly separated from the flow path of the outdoor air, the indoor air and outdoor air are not mixed.

In detail, the air sucked into the suction air suction part 120 is sucked only through the suction air suction part 280 of the total heat exchanger 200 having a rectangular cross section, and the air sucked through an exhaust suction part 130 is sucked only through an exhaust suction part 290 of the total heat exchanger 200 having a rectangular cross section. Therefore, since it is installed away from the flow path, a dead zone in which air does not flow well is not formed. In addition, since the channels in the total heat exchanger 200 are extended in the horizontal direction without any curves in the upper and lower directions, there is not airflow in the upper and lower directions, so that the resistances in the flow are not caused. In addition, in the present invention, since air does not flow in the multiple layers in the inlet and outlet portions of the total heat exchanger 200, the resistances in the flow of air is effectively prevented. Only the direction of the channel formation is changed at a certain height of the total heat exchanger, so that the same rectangular shapes are achieved.

Therefore, in the present invention, it is possible to more enhance the efficiency of heat exchange of the ventilator based on the decrease of the flow resistance of air and the removal of the dead zone. Even when the ventilator is fabricated with a small size, it is possible to achieve the same effects as a large capacity ventilator.

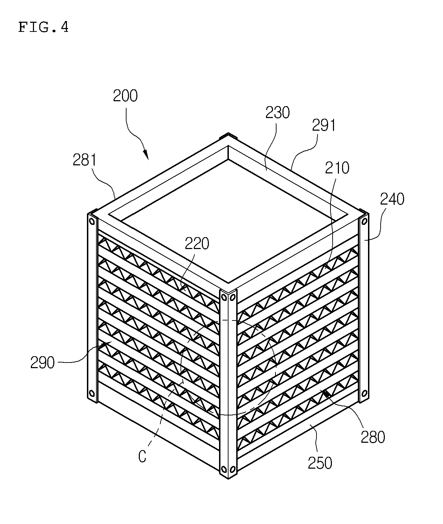

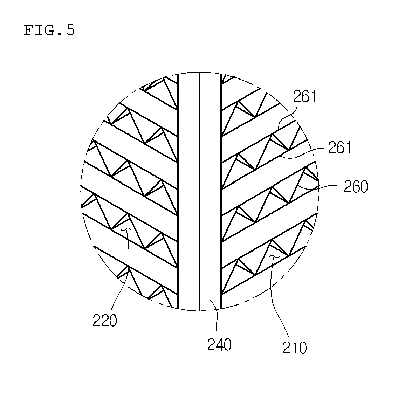

Figure 4 is a perspective view illustrating a total heat exchanger according to the present invention, and Figure 5 is an enlarged view of the portion C of Figure 4.

As shown in Figures 4 and 5, the total heat exchanger 200 according to the present invention has a hexahedral shape wherein air is sucked and discharged in a lateral direction. Namely, the air is sucked through the suction air suction part 280 and the exhaust suction part 290 formed in one side surface of the total heat exchanger 200, and the air is discharged through the opposite side of the same.

In detail, the total heat exchanger 200 includes a rectangular plate shaped upper frame 230, a lateral frame 240 that is attached to four corners of the upper frame 230 and is vertically extended, and a lower frame 250 of which corners are attached to the ends of the other side of the lateral frame 240. In addition, a suction channel 210 and an exhaust channel 220 are alternately stacked between the upper frame 230 and the lower frame 250 in the upper and lower directions. In details, the outdoor air is sucked and discharged through the suction channel 210, and the indoor air is sucked and discharged through the exhaust channel 220. The construction of the total heat exchanger 200 will be described. Since the upper and lower sides of the total heat exchanger 200 are blocked by the upper frame 230 and the lower frame 250, air is not sucked or discharged. In addition, the suction air suction part 280 and the exhaust suction part 290 are formed in four corners of the lateral side of the total heat exchanger 200, and a suction discharge part 281 and an exhaust discharge part 291 are formed in the opposite lateral sides of the suction air suction part 280 and the exhaust suction part 290.

Each channel 210 and 220 includes a heat exchange plate 261 in which a heat exchange is achieved between the outdoor air and indoor air based on a heat transfer operation, and a wrinkle plate 260 that is provided between the spaced-apart heat exchange plates 261 for thereby guiding the air to flow in a certain direction. Therefore, the heat exchange plate 261 is coupled to the upper side and lower side of the wrinkle plate 260.

The wrinkle plate 260 allows the air to uniformly flow in the channels 210 and 220, so that the heat exchange is enhanced during the flow of air. The wrinkle plate 260 operates as fins capable of increasing the area of heat exchange, so that the heat exchange efficiency of the total heat exchanger is significantly enhanced. In detail, the wrinkle plate 260 is callable of increasing the total heat area with air for thereby transferring more heat. As the outdoor air and indoor air pass through the interiors of the channels 210 and 220, the heat exchange is performed by the heat exchange plate 261 and the wrinkle plate 260, so that the efficiency of the heat exchange of the total heat exchanger is more enhanced. The winkle plate 260 and/or the heat exchange plate 2651 are formed of a certain metal having a high heat transfer coefficient, for example, an aluminum material.

Here, the construction of the wrinkle plate 260 is not limited to the embodiment of the present invention. The wrinkle plate with various shapes and widths may be adapted. Namely, the wrinkle plate 260 may be designed in such a manner that the air is sucked through the suction parts 280 and 290 in the interiors of the channels, and the air is discharged through the discharge parts 281 and 291.

The suction channel 210 and the exhaust channel 220 are alternately stacked, so that the entrance for sucking the indoor air is crossed from the entrance for sucking the outdoor air. Therefore, the indoor air and outdoor air are crossed and flow in the interior of the total heat exchanger 200.

When the total heat exchanger 200 is mounted in the interior of the casing 110, it faces the bottom of the lower frame 250, and the upper frame faces the upper surface of the same. The lateral side frame 240 faces the front and rear sides and both sides of the ventilator 100.

Since the suction terminal and discharge terminal have the same height, a multiple layer flow section does not occur in the flows of indoor and outdoor air sucked by the total heat exchanger 200 having the above-described construction. Therefore, it is possible to prevent any loss due to the flow resistance occurring during the flow of air. Even though the capacities of the suction fans 140 and 150 are small, it is possible to obtain a high efficiency. The consumption of energy may be decreased. It is possible to obtain high heat exchange efficiency by a small sized ventilator.

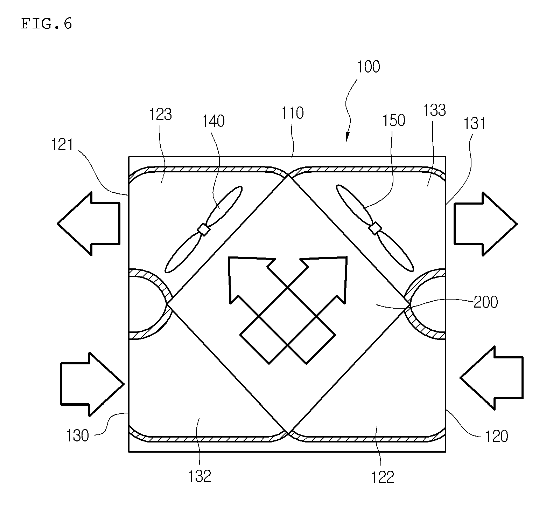

Figure 6 is a plane view illustrating the operation of the ventilator according to the present invention.

As shown in Figure 6, the ventilator 100 according to the present invention includes an exhaust flow path formed of the exhaust suction part 130, the exhaust suction duct 132, the total heat exchanger 200, the exhaust discharge duct 133, and the exhaust discharge part 131. In addition, there is provided a suction flow path formed of the suction part 120, the suction duct 122, the total heat exchanger 200, the suction discharge duct 123 and the suction discharge part 121. The suction flow path crosses with the exhaust flow path in a X shape.

In more detail, the suction air suction part 120 and the exhaust suction part 130 are the same horizontal line, and the discharge part 121 and the exhaust discharge part 131 are the same horizontal line by channel. Since the air flow path is formed like that, the length of the air flow is decreased, and the loss in flow is more decreased.

### [Second embodiment]

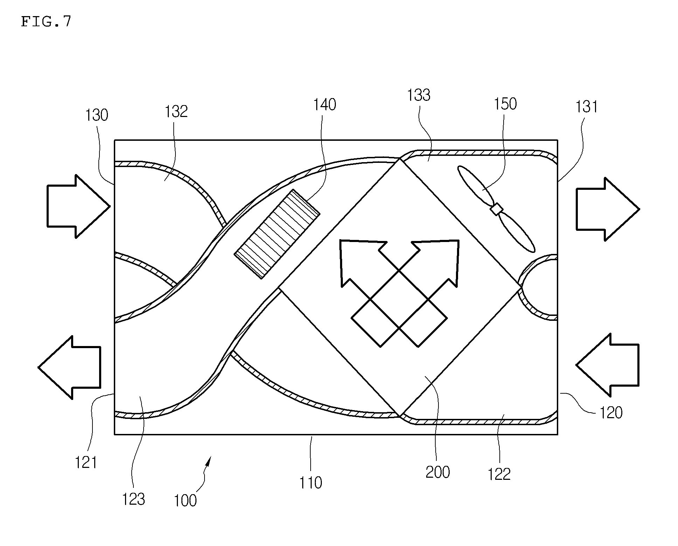

Figure 7 is a plane view of a ventilator according to a second embodiment of the present invention. Since the second embodiment of the present invention is the same as the first embodiment of the present invention except for the construction of the duct, the detailed description will be omitted.

As shown in Figure 7, the suction air discharge duct 123 and the exhaust suction duct 132 are crossed at a vertically distanced portion and are connected with the total heat exchanger 200. The suction air discharge duct 123 and the exhaust suction duct 132 are crossed in the interior of the ventilator 100 based on the characteristic of the fan. In the case that the suction fan 140 is a centrifugal fan like sirocco fan or turbo fan in which air is sucked in an axial direction and discharged in a radius direction, it is needed to minimize the resistance in air flow discharged from the suction fan 140. In other words, since the direction of the airflow in the centrifugal fan is curved at 90° at the sides of inlet and outlet of the fan, it is needed to curve the direction of the flow path for thereby decreasing the resistance of airflow.

When the flow path like in the first embodiment of the present invention is formed in a state that the centrifugal fan, the direction of the flow of the air discharged in a state that the direction of flow is curved at 90° by the centrifugal fan should be curved gain in the straight line direction. In addition, when the flow path is changed, a lot of resistance occurs based on the inner shape of the duct, resulting a lot of noses.

In the present invention, the suction air discharge duct 123 and the exhaust suction duct 132 are overlapped at the upper and lower positions, but the flow paths of the same are not mixed. In the detailed embodiment of the present invention, when the centrifugal fan is used, the neighboring ducts should be obviously crossed with each other.

### [Third embodiment]

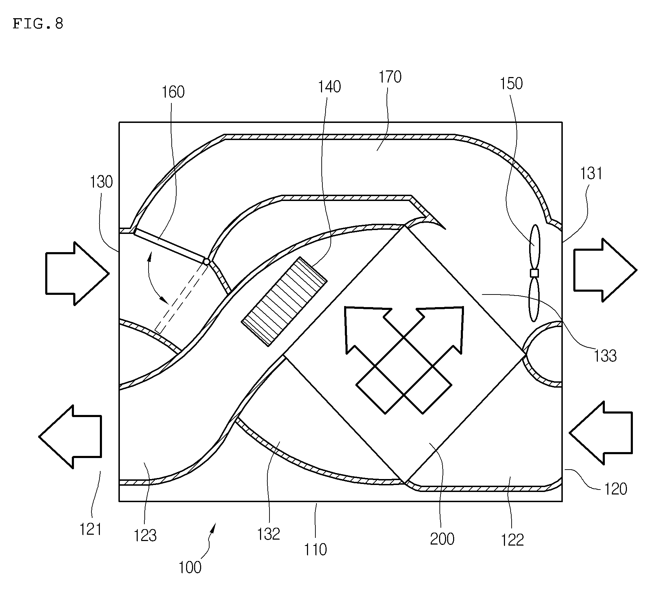

Figure 8 is a plane view of a ventilator according to a third embodiment of the present invention. The third embodiment of the present invention is the same as the second embodiment of the present invention except for the construction of a switching unit provided in the flow path. Therefore, the detailed description will be omitted except for the above different construction.

In the third embodiment of the present invention, the ventilator 100 further includes a ventilation duct 170 connected with the exhaust suction part 130 and the exhaust discharge part 131. In addition, a flow path switching unit 160 is further provided at an entrance of the exhaust suction part 130. In particular, the flow path switching unit is preferably provided near the exhaust suction part 130. If it is installed near the exhaust fan 150, the installation may be complicated. In addition, a certain problem may occur in the operation of the flow path switching unit 160 due to a turbulent flow near the exhaust fan 150. The exhaust fan 150 may be installed in any portion in the flow path in which the ventilation duct 170 is divided for thereby achieving a desired operation in the present invention. The flow path switching unit may includes a conventional damper, and a certain device may be adapted wherein it is rotatable with respect to a hinge point by a certain driving device.

The operation of the preferred embodiment of the present invention will be described. The ventilator according to the present invention may be operated in the total heat exchange mode or the conventional ventilation operation mode.

First, in the total heat exchange mode, the flow path switching unit 160 blocks the entrance of the ventilation duct 170, so that the indoor air flows through the total heat exchanger 200. At this time, since the heat exchange is performed by the total heat exchanger 200, the indoor temperature and moisture are constantly maintained together with the function of ventilation.

In the case that the ventilation operation is performed without the total heat exchange, since the flow path switching unit 160 blocks the entrance of the exhaust suction duct 132, the indoor air is discharged through the ventilation duct 170. In a preferred embodiment of the present invention, a blower may be installed in the interior of the ventilation duct 170 for thereby achieving fast ventilation.

### [Fourth embodiment]

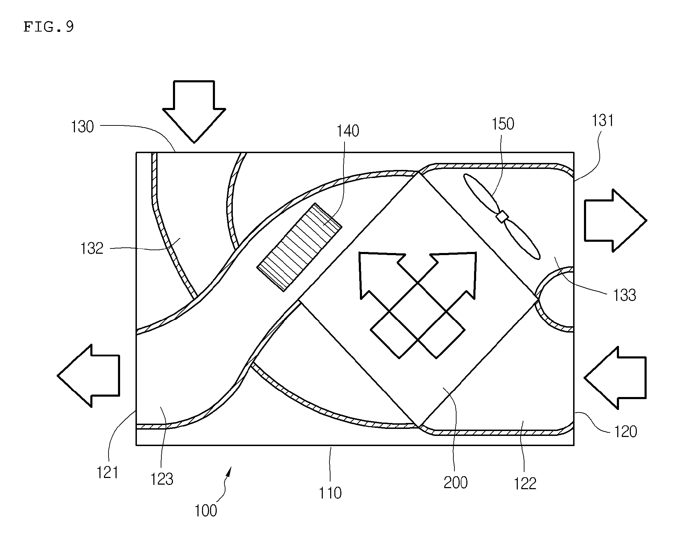

Figure 9 is a plane view illustrating a ventilator according to a fourth embodiment of the present invention. The fourth embodiment of the present invention is the same as the second embodiment of the present invention except for the construction that the exhaust suction part is not on the same straight line as the exhaust discharge part, but is at the different position. The constructions not described in detail may be deemed similar with the constructions of the first and second embodiment of the present invention.

As shown in Figure 8, in the ventilator 100 according to the present invention, the installation position of the exhaust suction part 130 for sucking the indoor air is not parallel with the exhaust discharge part 131, but is formed at a certain angle. In other words, the flow direction of the air sucked through the exhaust suction part 130 and the flow direction of the air discharged through the exhaust discharge part 131 are not parallel. Namely, the exhaust suction part 130 is formed in at a different position, so that the connection with a certain duct connected with the exhaust suction part 130 is easily achieved. When forming connection terminals, other preferred methods and positions may be easily adapted, so that the convenience of use is enhanced.

The construction that the flow direction of the air is not parallel is not limited to the construction of the exhaust suction part 130. Namely, other suction parts and/or discharge parts may be installed with various directions based on the position that the ventilator 100 is installed.

In the first, second and third embodiments of the present invention, the suction air suction part and the suction air discharge part are installed on the same straight line in parallel, and the exhaust suction part and the exhaust discharge part are installed on the same straight line in parallel. However, in the fourth embodiment of the present invention, the suction part and the discharge part may be installed in the other directions. With the above construction, there may not be limits in the formation directions and positions of any types of ducts. Therefore, it is possible to further enhance the convenience of use.

In the present invention, it is possible to make the ventilator smaller. The construction of flow structure is enhanced, for thereby enhancing a heat exchange efficiency.

In addition, since the flow structure is improved, the dead zone in which heat exchange is not efficiently performed is minimized. A multiple layer flow section does not occur in the flow path, thus decreasing the resistance in airflow.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. A ventilator, comprising:
a casing;
a flow path that is formed in the interior of the casing wherein indoor air and/or outdoor air are sucked in and discharged through the flow path; and
a total heat exchanger connected to the flow path, and including a plurality of channels which are extended horizontally, and the indoor and/or outdoor air flows through the channel horizontally.

2. The ventilator of claim 1, wherein a blower is installed in said flow path.

3. The ventilator of claim 1, wherein said channels are vertically stacked.

4. The ventilator of claim 1, wherein fins are provided in the inside of the channels for enhancing the heat exchange efficiency.

5. The ventilator of claim 1, further providing a plurality of wrinkled plates that divide the channels into horizontal directions.

6. The ventilator of claim 1, wherein at least one of the suction part and the discharge part of the outdoor air and/or indoor air is separately installed in one side surface of the casing.

7. The ventilator of claim 1, wherein said flow path includes at least one ventilation duct that is not connected with the total heat exchanger, and a flow path switch means which performs the switching function of another duct connected to the total heat exchanger and the ventilation duct.

8. The ventilator of claim 1, wherein said suction duct and discharge duct are crossed at at least one point.

9. The ventilator of claim 1, wherein at least one centrifugal fan is formed in the flow path, and a duct having the centrifugal fan is crossed with respect to another duct to decrease a resistance in the flow of air from the centrifugal fan.

10. The ventilator of claim 1, wherein the upper and lower surfaces of the total heat exchanger are blocked

11. The ventilator of one among claims 1 through 10, wherein a horizontal section and cross section of the total heat exchanger at a certain height are the same size formed in rectangular shapes.
